# EUROPÄISCHE PATENTSCHRIFT

(11) **EP 1 593 345 B1**
(45) Veröffentlichungstag und Bekanntmachung des Hinweises auf die Patenterteilung: **21.02.2007**
(21) Anmeldenummer: 04010870.6
(22) Anmeldetag: 07.05.2004
(51) Int. Cl.: A61B 5/20, G01F 3/38

(54) **Vorrichtung zur automatischen Überwachung des Flusses einer Flüssigkeit, insbesondere Urin**
Automatic liquid flow monitor, especially for urine
Appareil de contrôle automatique du débit d'un fluide, en particulier d'urine

(43) Veröffentlichungstag der Anmeldung: 09.11.2005
(73) Patentinhaber: Transmed Medizintechnik GmbH & Co. KG, 33181 Bad Wünnenberg (DE)
(72) Erfinder: Pelster, Michael, 59581 Warstein-Hirschberg (DE)
(74) Vertreter: Grosse, Wolf-Dietrich Rüdiger

(56) Entgegenhaltungen:
- EP-A- 0 109 373
- EP-B- 0 073 156
- DE-A- 3 411 449
- US-A- 5 269 443

## Beschreibung

Die Erfindung betrifft eine Vorrichtung zur automatischen Überwachung des Flusses einer unregelmäßig austretenden Flüssigkeit, insbesondere des aus einem Katheter austretenden Urinflusses, nach den Merkmalen des Oberbegriffs des Anspruchs.

Um den Flüssigkeitshaushalt eines katheterisierten Intensivpatienten ermitteln zu können, ist es unter anderem notwendig, das ausgeschiedene Urinvolumen zu bestimmten Zeitpunkten, zum Beispiel zu jeder vollen Stunde, zu bestimmen.

Bekannte, nicht-elektrische Messsysteme nehmen eine solche Bestimmung mit dem Messbecher-Prinzip vor. Dabei wird das Volumen des in einen skalierten Kunststoff-Messbecher fließenden Urins durch das Pflegepersonal abgelesen und der Urin anschließend vollständig in einen mit dem Messbecher verbundenen Einweg-Sammelbeutel umgefüllt.

Des weiteren sind nach dem Stand der Technik zur Überwachung Füllstandsmesser bekannt, bei denen durch Kapazitätsmessung oder durch Wiegen der Füllstand des Urins in einer Messkammer bestimmt wird. Bei der Gewichtsbestimmung ist aber problematisch, dass die Dichte des Urins zum Teil erheblich schwankt (1,002 bis 1,06 g/ml). Bedingt wird dies insbesondere durch Festkörper wie Blutkoagel oder Fettsteine, die mit dem Urin ausgeschwemmt werden. Aus einer reinen Gewichtsmessung des Urins kann somit nicht fehlerfrei auf das Volumen des Urins geschlossen werden.

Daneben ist es problematisch, dass der Urinfluss in der Regel schubweise erfolgt. Der Zuleitungsschlauch zwischen dem Urinkatheter und dem Messsystem liegt in der Regel wenigstens teilweise horizontal auf dem Patientenbett, so dass sich der Urin im Schlauch ansammelt und schubweise in das Messsystem fließt. Ein üblicher Zuleitungsschlauch mit einem Innendurchmesser von ca. 7 mm und einer Länge von 1,2 m fasst bis zu 46 ml Urin. Insbesondere dann, wenn sich der Patient bewegt bzw. seine Lageposition ändert, können große Urinmengen sehr schnell in einem Schwall in das Messsystem strömen.

Häufig wird auch die gesamte Liegefläche des Patientenbetts nach vorne oder nach hinten gekippt. Das mit dem Bett verbundene Messsystem nimmt dann ebenfalls eine Schräglage an, was sich ggfs. auf die Messgenauigkeit negativ auswirkt.

Diese Probleme löst die Messung des Urinvolumens mit Hilfe einer automatischen Schlauchpumpe, wie dies in den Dokumenten DE 3411449 A1 und EP 0073156 B1 beschrieben ist.

Bei der DE 34 11 449 A1 wird ein mit einem Katheter verbundener Abflussschlauch durch eine Schlauchpumpe bzw. Rollenpumpe geführt und von dort in einen Auffangbeutel, wobei die Schlauchpumpe so gesteuert wird, dass der Flüssigkeitsstand an einer vorbestimmten Marke am Schlauchpumpeneingang konstant gehalten wird und die durchgeschleuste Flüssigkeitsmenge durch Zählung der Umdrehungen oder Messung des Drehwinkels der Pumpe gemessen wird. Die Urin-Erkennung in Form von Elektroden ist oberhalb der Schlauchpumpe und diese wiederum oberhalb des Auffangbeutels angeordnet, so dass das Gesamtsystem relativ hoch ist.

Üblicherweise wird das Messsystem am Patientenbett, zumeist seitlich im Fußbereich, am Rahmengestell angebracht. Da Patienten zum Teil ausgesprochen nah zum Boden gelagert werden, ist das resultierende Höhenmaß dieses bekannten Systems problematisch.

Zudem bildet Urin Ablagerungen an allen Kontaktflächen (Schläuche, Sammelbehältnisse etc.). Werden Elektroden, wie bei der DE 34 11 449 A1, in ein Sammelbehältnis eingebracht, um durch den Kontakt zum Urin den Füllstand zu ermitteln, kann sich mit der Zeit zwischen dem Urin und den Elektroden eine hochohmige Schicht aus Urinablagerungen bilden, die zu einer Beeinträchtigung oder Aufhebung der Messfunktion führt. Auch können Teile des Messsystems, die im Zuge der Volumenmessung bewegt werden müssen und dabei mit Urin in Kontakt treten, in ihrer Beweglichkeit eingeschränkt oder gestört werden.

Nach dem gleichen, aus der DE 34 11 449 A1 bekannten, Prinzip arbeitet auch das aus der EP 0 073 156 B1 offenbarte Gerät zum automatischen Messen und Sammeln des Urinausflusses eines Patienten. Der Urin fließt über einen Zuleitungsschlauch in eine Messsäule bzw. -kammer, die von mindestens einem Füllstandssensor überwacht wird. Am unteren Ende der Messkammer ist ein nach unten ragender Pumpenschlauch angeschlossen, der durch eine Schlauchpumpe geführt wird und in einem Sammelbeutel endet. Die Schlauchpumpe quetscht den Pumpenschlauch luft- und wasserdicht ab, so dass der Urin nur bei Bewegung der Pumpe durch den Schlauch fließen kann. Übersteigt das Urinvolumen in der Messkammer einen definierten Füllstand, pumpt die Schlauchpumpe den Urin mit einer definierten Fördermenge ab, bis dass der Füllstand in der Messkammer wieder unterschritten wird. Aus Fördermenge und Pumpdauer lässt sich das abgepumpte Urinvolumen bestimmen.

Bei dieser bekannten Messanordnung ist nachteilig, dass der Pumpenschlauch am unteren Ende der Messkammer angeschlossen wird und von dort aus zum oben liegenden Sammelbeutel-Zugang geführt werden muss. Der Schlauch ist somit lang, wodurch er ungünstig zu handhaben ist, hohe Kosten hervorruft und Fehler- bzw. Gefahrenpotentiale verursacht. So kann er bei falschem Einlegen abknicken oder eine Schlaufe bilden, in die sich beim Transport des Messsystems Gegenstände verhaken und den Schlauch abreißen können. Stöße und Schläge gegen das Messsystem sind durchaus üblich. Das Verfangen von vorstehenden Schlaufen, Haken, Schnüren etc. in Gestelle herumstehender Betten, Tische o. ä. führt in der klinischen Praxis regelmäßig zu Beschädigungen.

Ausgehend hiervon liegt der Erfindung die Aufgabe zugrunde, eine gattungsgemäße Vorrichtung dahingehend weiterzuentwickeln, dass die Vorrichtung ein bestimmtes Höhenmaß nicht überschreitet, im Handling leicht ist und keine Gefahrenpotentiale beim Gebrauch aufweist.

Nach der Erfindung wird diese Aufgabe dadurch gelöst, dass zum einen die Messkammer seitlich neben dem Sammelbehältnis in etwa gleicher Höhe mit diesem angeordnet ist. Zum anderen ist die Messkammer mit einem Steigrohr versehen. Dieses ist am unteren Ende offen, um die in der Messkammer befindliche Flüssigkeit aufzunehmen, und am oberen Ende mit einem in das Sammelbehältnis mündenden Schlauch verbunden, wobei der Schlauch die zwischen dem oberen Steigrohrende und dem Sammelbehältnis angeordnete Schlauchpumpe durchläuft.

Die Messkammer wird demnach nicht oberhalb des Sammelbeutels angeordnet, sondern neben diesem, d.h. seitlich oder ggfs. dahinter. Auf diese Weise entspricht die maximale Höhe der Sammeleinrichtung dem Höhenmaß eines einfachen Urinsammelbeutel-Systems. Die Vorrichtung kann demnach auch bei tief gelagerten Patienten gut angeordnet werden. Bei einer seitlichen Neben-Anordnung resultiert die maximale Tiefe des Messsystems aus den Maßen eines befüllten Sammelbeutels. Für das Messsystem ist hinsichtlich der Breite keine Beschränkung vorgegeben.

Außerdem wird ein Schutz vor Beschädigungen der Sammeleinrichtung erreicht. Der Pumpenschlauch ist durch die Integration des Steigrohrs in die Messkammer sehr kurz und wird auf direktem Wege durch die Schlauchpumpe in den Sammelbeutel geführt. Die beschriebenen Nachteile, die aus einem zu langen Pumpenschlauch resultieren, der vom unteren Ende der Messkammer zur oben liegenden Eintrittsstelle in den Sammelbeutel geführt wird, kann erfindungsgemäß durch die Integration des Steigrohrs in das Pufferreservoir der Messkammer verhindert werden. Daraus ergeben sich folgende signifikante Vorteile:

Erstens liegt eine vereinfachte Handhabung vor. Sämtliche Zu- und Abgänge der Messkammer befinden sich im oberen Bereich der Messkammer. Dadurch kann die Messkammer wie eine Art "Patrone" leicht in eine speziell ausgeformte Öffnung im Gehäuse der Steuereinheit eingeführt und dort arretiert werden.

Des weiteren können die Abgänge vom Pufferreservoir der Messkammer, d.h. der ein Überlaufschlauch und der Pumpenschlauch, derartig positioniert sein, dass ein Vertauschen der Schläuche unmöglich ist. Ein Schlauchadapter für den Pumpenschlauch lässt sich vorteilhafterweise so ausrichten, dass sich der Pumpenschlauch direkt vor dem Eingang des Pumpenkopfs befindet und für den Anwender somit offensichtlich ist, dass dieser Schlauch in die Schlauchpumpe einzulegen ist. Vor dem Hintergrund einer wachsenden Belastung des Pflegepersonals infolge einer Personalreduzierung in den Krankenhäusern ist die einfache Handhabung auch in Phasen verminderter Konzentrationsfähigkeit (Stresssituationen, Nachtdienst) von zunehmender Bedeutung.

Zudem bietet die vorgeschlagene Vorrichtung einen Schutz vor Störeinflüssen auf die Messsensorik. In Abhängigkeit der "Patronenform" der Messkammer mit den oben positionierten Zu- und Abgängen kann die Messkammer-Aufnahme im Gehäuse der Steuereinheit so konstruiert sein, dass diese Aufnahme lediglich oben eine Öffnung zum Einführen der Messkammer aufweist. Insbesondere der untere Teil der Messkammer mit der angrenzenden, sensiblen Messsensorik zur Urin-Luft-Erkennung lässt sich somit mittels die Messkammer umschließender Wandungen gegen Störeinflüsse (Licht, elektromagnetische Felder etc.) abschirmen.

Zudem erreicht die Erfindung eine Kostenreduzierung. Das Schlauchmaterial, das für den Pumpenschlauch verwendet werden sollte, muss ausgesprochen hochwertig sein, da es komplexe Anforderungen erfüllen muß. Grund hierfür ist, dass die Förderkonstante über die gesamte Anwendungsdauer des Schlauchs (bis zu 14 Tage im Dauerbetrieb) konstant sein muss. Dies setzt voraus, dass der Schlauch sowohl formstabil als auch elastisch ist. Wird er zwischen Pumpenkopfwandung und Pumpenrolle eingequetscht, muss er sich stark verformen können. Dreht sich die Pumpenrolle weiter, muss er sich sofort wieder auf seinen ursprünglichen Querschnitt ausdehnen. Zusätzlich ist auszuschließen, dass sich Urin an den Innenflächen ablagert und den Innendurchmesser verringert. Es empfiehlt sich, für solche Anwendungen Silikonschläuche zu verwenden, wobei der Silikon durch Beimischung bestimmter Bestandteile, zum Beispiel Platin, veredelt wird. Die Innenfläche ist zudem zum Beispiel mit Teflon beschichtet, um Anhaftung von Urin zu verhindern.

Derartige Schläuche sind in der Fertigung sehr aufwendig und daher teuer in der Anschaffung. Durch die Integration des Steigrohrs in die Messkammer wird die Länge des Pumpenschlauchs erheblich reduziert (um ca. 50-75% zum Stand der Technik), so dass sich die Kosten für das Verbrauchsmaterial erheblich reduzieren.

Nach einer Weiterentwicklung der Erfindung soll ein zweiter Füllstand im Pufferreservoir der Messkammer mit Hilfe eines zusätzlichen Füllstandssensors detektiert werden. Dieser ist auf einem höheren Füllstandsniveau positioniert, beispielsweise 20 ml zu 3ml. Der Füllstand wird dann auf diesem höheren Niveau konstant gehalten.

Das Pufferreservoir kann vorteilhafterweise farblos und transparent ausgefertigt sein. Dann kann der Urin in Farbe und Konsistenz geprüft werden (Sichtkontrolle).

Zudem kann zu bestimmten Zeitpunkten, zum Beispiel zu jeder vollen Stunde, das Pufferreservoir bis zur unteren Füllstandsmarke (3 ml) abgepumpt werden, um das ausgeschiedene Urinvolumen pro Stunde genau angeben zu können.

Nach einer Weiterentwicklung der Erfindung ist die Vorrichtung mit einem Pumpenkopf mit einer heb- und senkbaren Pumpenkopfwandung ausgebildet. Auf diese Weise lässt sich der Pumpenschlauch flexibel in den Pumpenkopf einlegen und wieder entfernen.

Weitere Einzelheiten und Vorteile der Erfindung ergeben sich aus der nachfolgenden Beschreibung, in der die in den Figuren dargestellte Ausführungsform der Erfindung näher erläutert wird. Es zeigen:
- Fig. 1: eine Halbschnittdarstellung der Messkammer mit angeschlossenen Schläuchen und den Funktionselementen "Urin/Luft-Erkennung" (Füllstandssensor) und Schlauchpumpe des elektronischen Steuergeräts;
- Fig. 2: eine Prinzipskizze des Pumpenkopfs der Schlauchpumpe.

Die Vorrichtung nach der Erfindung bzw. das Messsystem besteht aus einer Steuereinrichtung 1 bzw. einem Steuergerät und einem Verbrauchsartikel bzw. Sammeleinrichtung 2. Der Verbrauchsartikel 2 setzt sich aus den Hauptelementen Messkammer 3, Pumpenschlauch 4 und einem Sammelbehältnis 5 bzw. Sammelbeutel zusammen.

Aus Sicherheitsaspekten kann zusätzlich ein Überlaufschlauch 6 vorgesehen sein, der die Messkammer 3 direkt mit dem Sammelbeutel 5 verbindet.

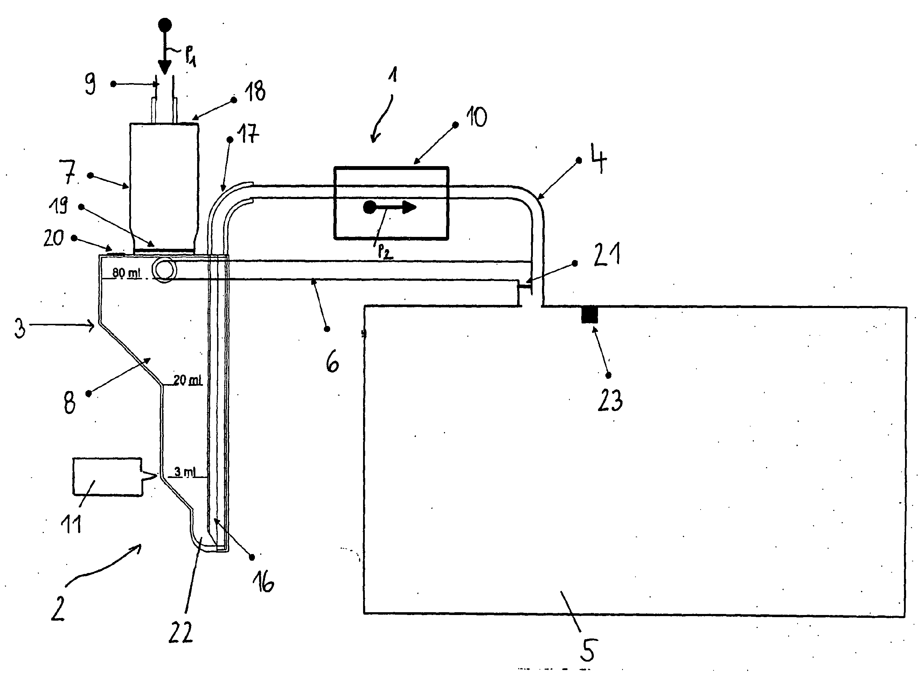

Die Messkammer 3 setzt sich aus den Teilkomponenten Tropfkammer 7 und Pufferreservoir 8 zusammen. Am vorderen Ende eines Zulaufschlauchs 9 ist eine Katheterkupplung zur Verbindung mit einem Harnkatheter angebracht (in Fig. 1 nicht gezeigt). Der Urin-Zulauf vom Katheter ist mit dem Pfeil P₁ bezeichnet. Der Verbrauchsartikel 2 wird an der Steuereinrichtung 2 bzw. dem Steuergerät befestigt, das im Wesentlichen die Funktionselemente Schlauchpumpe 10 und Sensorik für die Urin/Luft-Erkennung 11 beinhaltet.

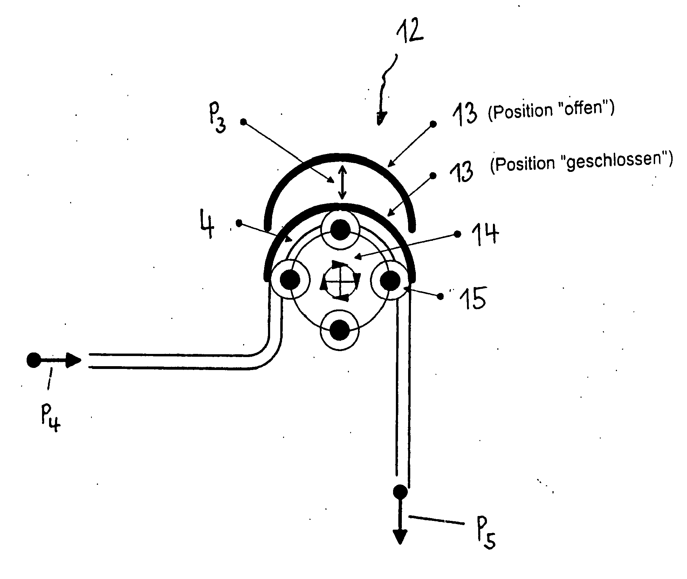

Die Schlauchpumpe 10 setzt sich aus einer Antriebseinheit (Motor-Getriebeeinheit), einem Pumpenkopf 12 und einem Encoder für die Überwachung der Drehbewegung zusammen. Der Pumpenkopf 12 besteht gemäß Fig. 2 aus einer beweglichen äußeren Pumpenkopfwandung 13 und einem Rotor 14. Der Rotor 14 ist mittig drehbar gelagert und wird von der Antriebseinheit angetrieben. Im Abstand von 90° sind am Rotor 14 Pumpenrollen 15 drehbar befestigt.

Die Pumpenkopfwandung 13 lässt sich mittels Parallelführung per Hand in die Position "offen" bewegen. Die Bewegungsrichtung der Pumpenkopfwandung 13 ist mit P₃ bezeichnet. Dadurch lässt sich der Pumpenschlauch 4 leicht aus dem Pumpenkopf 12 entnehmen bzw. einlegen. Wird die Pumpenkopfwandung 13 bei eingelegtem Schlauch 4 in die Position "geschlossen" bewegt, rastet die Pumpenkopfwandung 13 in ihre Endposition ein. Dadurch wird der Pumpenschlauch 4 zwischen mindestens einer Pumpenkopfrolle 15 und der Pumpenkopfwandung 13 luft- und wasserdicht abgequetscht.

Durch den Encoder wird die Drehbewegung des Pumpenkopfs 12 in 180°-Schritten detektiert. Das pro 180°-Drehung geförderte Urinvolumen ist genau definiert (beispielsweise Förderkonstante k= 0,90 ml/180°). Wird der Pumpenkopf 12 in Bewegung gesetzt, kann die Drehbewegung erst angehalten werden, wenn eine volle 180°-Drehung abgeschlossen ist. Somit startet und endet der Pumpenkopf ausschließlich in 2 Drehpositionen, die um 180° versetzt sind. Dadurch ist das geförderte Urinvolumen grundsätzlich ein ganzzahliges Vielfaches der Förderkonstante k. Mit P₄ ist der Urin-Zulauf aus dem Pufferreservoir 8, mit P₅ der Urin-Ablauf zum Sammelbeutel 5 bezeichnet.

Ein wesentlicher Bestandteil des Pufferreservoirs 8 ist das Steigrohr 16, das unten bzw. an seinem unteren Ende offen ist, um vom Grund des Pufferreservoirs 8 den Urin abzupumpen, und das am oberen Ende in einen Schlauchadapter 17 übergeht (vgl. Fig. 1). An dem Schlauchadapter 17, der ggf als 90°-Bogen ausgeführt ist, ist der Pumpenschlauch 4 befestigt, der wiederum am Zugang zum Sammelbeutel 5 endet. Das Steigrohr 16 kann sowohl im Innern des Pufferreservoirs 8 eingesetzt als auch von außen fest am Pufferreservoir 8 angebracht sein. Bei der gezeigten Ausführungsform, auf die die Erfindung nicht beschränkt ist, ist das Steigrohr 16 an der dem Sammelbehältnis zugewandten Seite im Innern des Reservoirs 8 angeordnet.

Es ergibt sich der wesentliche Vorteil, dass sämtliche Schläuche im oberen Bereich der Messkammer 3 angebracht sind und das Pufferreservoir 8 einschließlich Steigrohr 16 eine definierte Außenkontur bildet, mit der es in eine speziell geformte Aufnahme im Steuergerät 1 eingesetzt werden kann.

An dem Pufferreservoir 8 kann knapp unterhalb der Tropfkammer 7 ein Überlaufschlauch 6 befestigt sein, der im Falle eines unzulässigen Aufstauens von Urin in dem Pufferreservoir 8 den direkten Urinabfluss in den Sammelbeutel 5 sicherstellt.

Der Sensor 11 für die Füllstandsmessung im Pufferreservoir 8 ist derartig ausgelegt, dass für die eindeutige Detektion von Urin bzw. Luft weder ein direkter Kontakt zu diesen Medien noch ein direkter Kontakt zum Pufferreservoir 8 bestehen muss. Er ist am Steuergerät 1 fest montiert und hat eine definierte Position zum Pufferreservoir 8. Entweder ist es ein Sensor, der von einer Seite (tastend) Urin bzw. Luft detektiert, oder eine Sensoreinheit, die aus Sender und Empfänger besteht und nach dem Lichtschrankenprinzip funktioniert. Sender und Empfänger stehen hierzu in Sichtkontakt, das Pufferreservoir 8 befindet sich dazwischen. Eine Laserdiode im Sender strahlt Licht aus, dessen Wellenlänge auf die Schwingfrequenz der Wassermoleküle abgestimmt ist. Befindet sich eine wässrige Lösung zwischen der Laserdiode und der Photo-Diode des Empfängers, wird das Licht von Wassermolekülen absorbiert und erreicht die Photo-Diode nicht bzw. signifikant abgeschwächt.

Nachfolgend wird die Funktion der Vorrichtung beschrieben. Der Urin strömt durch den Zulaufschlauch 9 in die Tropfkammer 7, die als pasteursche Kammer dient und durch eine Belüftungsmembran 18 zwecks Druckausgleichs belüftet ist. Am Übergang zum Pufferreservoir 8 befindet sich ein Rückschlagventil bzw. Rückstrom-Sperrventil 19, durch das der Urin in diese Richtung ungehindert in das Pufferreservoir 8 fließen kann. In dem Pufferreservoir 8, das ebenfalls eine weitere Belüftungsmembran 20 aufweist, staut sich der Urin auf. Übersteigt der Füllstand eine definierte Füllstandsmarke (hier beispielsweise 3 ml), wird dies vom Füllstandssensor 11 erkannt und ein Sensorsignal an eine Schaltelektronik mit integrierten Mikroprozessoren übertragen. Die Schaltelektronik schaltet die Schlauchpumpe 10 ein.

Infolge der Drehbewegung der Schlauchpumpe 10 wird der Pumpenschlauch 4 in Richtung Sammelbeutel 5 - ebenfalls mit einer Belüftungsmembran 23 ausgestattet - ausgestreift. Die Pumprichtung ist durch P₂ bezeichnet. Dadurch bewegt sich die Luft in Steigrohr 16, Schlauchadapter 17 und Pumpenschlauch 4 in Richtung Sammelbeutel 5. Es bildet sich ein Unterdruck im Steigrohr 16 aus, durch den der Urin in das Steigrohr 16 gesaugt und anschließend über den Pumpenschlauch 4 weiter in den Sammelbeutel 5 gefördert wird (Saugheber-Effekt). Die Schlauchpumpe 10 wird angehalten, wenn der Füllstandssensor 11 keinen Urin mehr detektiert und eine volle 180°-Drehung des Pumpenkopfs 12 abgeschlossen ist. Die Anzahl der 180°-Drehungen des Pumpenkopfs 12 wird mit der Förderkonstante k multipliziert und dieser Wert zu den Volumina der vorherigen Abpumpzyklen hinzuaddiert. Die Summe wird in einem Display angezeigt.

Ist die Schlauchpumpe 10 defekt oder die Energieversorgung des elektrischen Messsystems unterbrochen, ist ein Abpumpen des Urins mit der Schlauchpumpe 10 nicht mehr möglich. In diesem Fall ist die Verbindung zwischen Messkammer 3 und Sammelbeutel 5 unterbrochen und es besteht die Gefahr, dass sich der Urin bis hin zum Patienten aufstaut und somit die Urinausscheidung des Patienten unterbrochen wird. Sofern eine automatische Fehlererkennung, beispielsweise mittels spezieller Überwachungssensorik nicht ausreichend zuverlässig ist oder sich Vorgaben aus Normen und Richtlinien auf diese Weise nicht erfüllen lassen, kann ein Überlaufschlauch 6 den ungehinderten Abfluss des aufgestauten Urins in den Sammelbeutel 5 gewährleisten. Am Ende des Überlaufsschlauchs 6 ist ein Rückstrom-Sperrventil 21 angeordnet.

Es ist zu beachten, dass das offene Ende des Steigrohrs 16 im Pufferreservoir 8 vollständig im Urin steht und beim Abpumpen keine Luft angesaugt wird. Diese Anforderung ist auch dann zu erfüllen, wenn sich das Messsystem samt Messkammer 3 in Schräglage befindet. Wie Fig. 1 zeigt, endet hierzu das offene Ende des Steigrohrs 16 in einer Vertiefung 22 des Pufferreservoirs 8. Diese Vertiefung 20 ist so gestaltet, dass bei einer Schräglage von bis zu 45° keine Luft in das Steigrohr eindringen kann. Vorteilhafterweise können Neigungssensoren vorgesehen sein, die die Neigung des Messsystems in beide horizontalen Raumrichtungen messen. Die Messwerte werden von der Schaltelektronik ausgewertet, die bei Überschreiten der 45°-Schräglage einen Alarm auslöst. Zusätzlich wird das Abpumpen von Urin unterbrochen.

Insgesamt wird eine Vorrichtung erhalten, die sich aus einem Einmalartikel und einem Investitionsgut, nämlich der Steuereinrichtung, zusammensetzt. Die Volumenbestimmung des Urins erfolgt automatisch zu exakt definierten Zeitpunkten. Das Personal muss nicht zu jeder vollen Stunde begonnene Arbeiten unterbrechen, um beispielsweise den Urinstand in Messbechern abzulesen. Die Volumenbestimmung erfolgt mit einem sehr geringen Messfehler, bei dem Ablesefehler durch das Pflegepersonal entfallen. Die Ausscheidungsraten der vergangenen Stunden und ggf. Tage eines Patienten können von dem elektronischen Messsystem gespeichert werden. Das Pflegepersonal kann die Daten zu jedem beliebigen Zeitpunkt am Steuergerät abfragen. Zudem können die erfassten Messwerte im Onlinebetrieb in eine Datenbank (Patientendaten-Management-System oder Fluid-Management-System) übertragen und automatisch ausgewertet werden. Die Handhabung des Systems ist sehr einfach, und der Schulungsaufwand für das Bedienpersonal kann auf ein Minimum reduziert werden. Zudem sind die Kosten für den Einmalartikel gering und können durch Funktion, Größe, Materialvielfalt und Fertigungsverfahren reduziert werden. Zudem arbeitet das System leise, so dass die Patienten hierdurch nicht zusätzlich belastet werden.

### Bezugszeichenliste:

- 1: Steuereinrichtung bzw. Steuergerät
- 2: Verbrauchsartikel
- 3: Messkammer
- 4: Pumpenschlauch
- 5: Sammelbehältnis bzw. Sammelbeutel
- 6: Überlaufschlauch
- 7: Tropfkammer
- 8: Pufferreservoir
- 9: Zulaufschlauch
- 10: Schlauchpumpe
- 11: Sensor für die Urin/Luft-Erkennung
- 12: Pumpenkopf
- 13: Pumpenkopfwandung
- 14: Rotor
- 15: Pumpenrollen
- 16: Steigrohr
- 17: Schlauchadapter
- 18: Belüftungsmembran
- 19: Rückschlagventil
- 20: Belüftungsmembran
- 21: Rückstrom-Sperrventil
- 22: Vertiefung im Pufferreservoir
- 23: Belüftungsmembran

## Patentansprüche

1. Vorrichtung zur automatischen Überwachung des Flusses einer unregelmäßig ausströmenden Flüssigkeit, insbesondere des aus einem Katheter austretenden Urinflusses, mit einer Sammeleinrichtung (2) mit einem Zulaufschlauch (9), der insbesondere mit einem Katheter verbunden ist, einer Messkammer (3), einem Pumpenschlauch (4) und einem Sammelbehältnis (5) sowie mit einer Steuereinrichtung (1) mit einer Schlauchpumpe (10) zur Entfernung des Urinausflusses aus der Messkammer (3) mit bekanntem Durchsatz,
**dadurch gekennzeichnet,**
a) **dass** die Messkammer (3) seitlich neben dem Sammelbehältnis (5) in etwa gleicher Höhe mit diesem angeordnet ist,
b) **dass** die Messkammer (3) mit einem Steigrohr (16) versehen ist, das am unteren Ende offen ist, um die in der Messkammer (3) befindliche Flüssigkeit aufzunehmen, und das am oberen Ende mit einem in das Sammelbehältnis (5) mündenden Schlauch (4) verbunden ist, wobei der Schlauch (4) die zwischen dem oberen Steigrohrende und dem Sammelbehältnis (5) angeordnete Schlauchpumpe (10) durchläuft.

## Claims

1. A device for automatically monitoring the flow of an irregularly escaping liquid, in particular the urine flow escaping from a catheter, comprising a collecting device (2) with a supply hose (9), which is connected, in particular, to a catheter, a measuring chamber (3), a pump hose (4), and a collecting vessel (5), and comprising a control device (1) with a hose pump (10) for removing the urine flow from the measuring chamber (3) with known flow rate,
**characterized in**
a) **that** the measuring chamber (3) is arranged laterally next to the collecting vessel (5) at approximately the same height with said collecting vessel (5),
b) **that** the measuring chamber (3) is provided with an uptake pipe (16), the lower end of which is open so as to accommodate the liquid located in the measuring chamber (3), and which, at the upper end, is connected to a hose (4), which discharges into the collecting vessel (5), whereby the hose (4) runs through the hose pump (10), which is located between the upper uptake pipe end and the collecting vessel (5).

## Revendications

1. Procédé pour la surveillance automatique du flux d'un liquide à écoulement irrégulier, notamment du flux d'urine sortant d'un cathéter, avec un dispositif collecteur (2), avec un flexible d'amenée (9), qui est relié notamment avec un cathéter, avec une chambre de mesure (3), un flexible de pompage (4) et un récipient collecteur (5), ainsi qu'avec un dispositif de commande (1) avec une pompe tubulaire (10) pour éliminer le flux d'urine de la chambre de mesure (3) avec un débit connu, **caractérisé en ce que**,
a) la chambre de mesure (3) est disposée à côté du récipient collecteur (5), environ à la même hauteur que celui-ci,
b) **en ce que** la chambre de mesure (3) est munie d'un tube ascendant (16) qui est ouvert sur l'extrémité inférieure, pour réceptionner le liquide qui se trouve dans la chambre de mesure (3) et qui sur l'extrémité supérieure est relié avec un flexible (4) débouchant dans le récipient collecteur (5), le flexible (4) traversant la pompe tubulaire (10) disposée entre l'extrémité supérieure du tube ascendant et le récipient collecteur (5).
